# EUROPÄISCHE PATENTANMELDUNG

(11) **EP 3 564 906 A1**
(43) Veröffentlichungstag der Anmeldung: **06.11.2019**
(21) Anmeldenummer: 18170877.7
(22) Anmeldetag: 04.05.2018
(51) Int. Cl.: G06T 11/00, G06T 7/11

(54) **VERFAHREN ZUR ERZEUGUNG VON BILDDATEN BEI EINEM COMPUTERTOMOGRAPHIEGERÄT, BILDERZEUGUNGSRECHNER, COMPUTERTOMOGRAPHIEGERÄT, COMPUTERPROGRAMMPRODUKT UND COMPUTERLESBARER DATENTRÄGER**

(71) Anmelder: Siemens Healthcare GmbH, 91052 Erlangen (DE)
(72) Erfinder: Ritter, André, 91054 Buckenhof (DE); Raupach, Rainer, 91336 Heroldsbach (DE)

(57) **Zusammenfassung**

Die Erfindung betrifft ein Verfahren zur Erzeugung von Bilddaten (BD) bei einem Computertomographiegerät (2), wobei CT-Rohdaten (RD) bereitgestellt werden, wobei ein Initialbild (IB) aus den CT-Rohdaten(RD) erstellt wird, wobei das Initialbild (IB) anhand anatomischer Merkmale (ME) in Bereiche (B) segmentiert wird, wobei für das Initialbild (IB) entsprechend der Bereiche (B) jeweils eine Bildmaske (MA) erstellt wird, welche einen Wirkungsbereich für eine jeweils zugeordnete Abbildungsvorschrift (V) definiert, wobei die Abbildungsvorschriften (V) in den durch die jeweiligen Bildmasken (MA) definierten Wirkungsbereichen angewendet werden, und wobei anhand des mittels der Abbildungsvorschriften (V) bearbeiteten Initialbildes (IB) die Bilddaten (BD) erzeugt werden.

## Beschreibung

Die Erfindung betrifft ein Verfahren zur Erzeugung von Bilddaten bei einem Computertomographiegerät. Des Weiteren betrifft die Erfindung einen Bilderzeugungsrechner mit einem Controller zur Durchführung des Verfahrens sowie ein Computertomographiegerät mit einem solchen Bilderzeugungsrechner.

Ein Computertomographiegerät (CT-Gerät) wird häufig zur Erzeugung von Bilddaten für die medizinische Diagnostik sowie für die Planung von Therapien eingesetzt. Hierzu werden aus mittels des CT-Gerätes aufgenommenen CT-Rohdaten die Bilddaten erzeugt, insbesondere rekonstruiert, und beispielsweise in Form einer Schnittdarstellung oder als eine dreidimensionale Volumendarstellung des untersuchten Objekts, typischerweise eines Patienten, genutzt. Dabei wird ein Betrag einer bestimmten Materialeigenschaft mittels (Bild-)Werte der Bilddaten dargestellt. Insbesondere ist jedem Pixel oder einem Voxel jeweils ein Bildwert zugeordnet. In der medizinischen Diagnostik entspricht dem jeweiligen Bildwert insbesondere ein Betrag eines Absorptionskoeffizienten, anhand dessen Strukturen im erfassten Bereich des Objekts unterschieden bzw. untersucht werden können.

Ferner werden solche Bilddaten in der Planung einer Strahlentherapie genutzt. Hierbei soll eine ortsaufgelöste Verteilung von Strahlendosen, welche dem Patienten im Zuge der Strahlentherapie zugeführt wird, möglichst genau vorherbestimmbar sein, um nicht malignes Gewebe zu schonen und/oder malignem Gewebe eine zur Therapie geeignete Strahlendosis zuzuführen. Erfolgt eine Bestrahlung beispielsweise mit Photonen, so erfolgt eine Wechselwirkung der eingebrachten Strahlung und dadurch bewirkter Sekundärprozesse insbesondere mit Elektronen. In geeigneter Weise wird für die Strahlentherapie als Bilddaten folglich eine Ladungsdichteverteilung, insbesondere eine Elektronendichteverteilung, herangezogen.

Die aus den CT-Rohdaten erzeugten Bilddaten können (Bild-) Artefakte, wie beispielsweise Strahlaufhärtungsartefakte, welche aufgrund einer Verwendung polychromatischer Röntgenstrahlung bei der Aufnahme der CT-Rohdaten auftreten, oder auch Sättigungsartefakte aufweisen. Diese Artefakte erschweren nachteilig eine zuverlässige Diagnose oder die Planung der Therapie, da der Bildwert nicht dem Betrag der Materialeigenschaft einer dem Bildwert zugeordneten (anatomischen) Struktur entspricht und infolge dessen die (anatomische) Struktur beispielsweise unscharf oder verzerrt dargestellt erscheint.

Es sind (Rekonstruktions-)Verfahren bekannt, welche einen Schwellenwert nutzen, um die Bilddaten zur korrigieren. Aus der DE 10 2015 225 395 A1 ist beispielsweise ein Verfahren zur Ermittlung einer räumlichen Verteilung eines Materialeigenschaftswerts bekannt. Hierin werden zunächst auf Basis von Mess-Projektionsdaten Bilddaten rekonstruiert. Anschließend werden Bildpunkte der Bilddaten unter Nutzung eines Schwellenwertes klassifiziert und entsprechend der Klassifizierung eine Verteilung von zwei Basismaterialien abgeschätzt. Mittels der abgeschätzten Verteilung und der Mess-Projektionsdaten wird eine räumliche Verteilung der Materialeigenschaftswerte ermittelt.

Nachteilig können bei Verfahren mit schwellenwertbasierter Korrektur unterschiedliche Materialien vergleichsweise schwer unterschieden werden, wenn diese durch ähnliche Bildwerte dargestellt sind und/oder wenn die unterschiedlichen Materialien eine überlappende Verteilung aufweisen. So kann beispielsweise Knochen und ein Kontrastmittle vergleichsweise schwer unterschieden werden. Des Weiteren entspricht beispielsweise aufgrund von Artefakten eine Verteilung der mittels Bildwerte repräsentierten Materialeigenschaften nicht der tatsächlichen Verteilung im Objekt. Basiert eine Korrektur der Bilddaten allerdings auf der Annahme, dass die segmentierten Bereiche der tatsächlichen Verteilung im Objekt entsprechen, können somit Fehler bei der Korrektur auftreten.

Der Erfindung liegt die Aufgabe zugrunde, eine Verbesserung der Qualität von aus CT-Rohdaten rekonstruierten Bilddaten zu ermöglichen. Insbesondere sollen dabei Artefakte vermieden oder zumindest reduziert werden.

Diese Aufgabe wird erfindungsgemäß gelöst durch ein Verfahren zur Erzeugung von Bilddaten mit den Merkmalen des Anspruchs 1. Des Weiteren wird diese Aufgabe erfindungsgemäß gelöst durch einen Bilderzeugungsrechner mit den Merkmalen des Anspruchs 9. Ferner wird diese Aufgabe erfindungsgemäß gelöst durch ein Computertomographiegerät mit den Merkmalen des Anspruchs 10.

Das erfinderische Verfahren dient der Erzeugung von Bilddaten bei einem Computertomographiegerät. Die Bilddaten weisen dabei (Bild-)Werte auf, welche insbesondere eine Materialeigenschaft repräsentieren. So sind die Bilddaten beispielsweise eine Darstellung der räumlichen Verteilung von Iod (Iodbild) als Kontrastmittel, eines das Absorptionsvermögen von Röntgenstrahlung repräsentierenden Absorptionskoeffizienten (Absorptionsbild) oder einer Ladungsdichte, beispielsweise der Kernladungszahl.

Verfahrensgemäß werden dabei zunächst CT-Rohdaten (CT-Aufnahmedaten) eines Untersuchungsbereichs, also des bei der Aufnahme der CT-Rohdaten erfassten Bereichs, eines zu untersuchenden Objekts, wie beispielsweise eines Patienten, bereitgestellt. Aus diesen CT-Rohdaten wird dann ein Initialbild erstellt, wobei hierzu beispielsweise eine aus dem Stand der Technik bekannte iterative Rekonstruktion oder vorzugsweise die sogenannte gefilterte Rückprojektion (filtered back projection) herangezogen wird.

Weiterhin wird das Initialbild anhand anatomischer Merkmale in Bereiche segmentiert und für jeden dieser Bereiche jeweils eine diesem Bereich entsprechende Bildmaske für das Initialbild erstellt. Ein anatomisches Merkmal ist dabei eine Eigenschaft einer (anatomischen) Struktur, wie beispielsweise ein Knochen, ein Organ, Gefäße, Nervenstränge oder ein Implantat, mittels welcher Eigenschaft diese Struktur von einer weiteren Struktur im Initialbild unterschieden werden kann und auch wird. Insbesondere ist diese Eigenschaft die Form (Gestalt), die Oberfläche, die Lage, die relative Lage dieser Struktur zur weiteren Struktur oder eine Strukturierung, wie beispielsweise spezifische Anordnung von Gefäßen oder Drüsen, der Struktur. Beispielsweise wird zur Segmentierung zusätzlich zu den anatomischen Merkmalen, insbesondere der Form oder der Lage der entsprechenden Struktur, ein Schwellenwert herangezogen. Insbesondere sind jedoch solche Strukturen voneinander auch unterscheidbar, wenn sich deren jeweilige Absorptionskoeffizienten, unter Berücksichtigung eines Bildrauschens, ausreichend unterscheiden. Hierbei ist also die Kenntnis eines (absoluten) Betrages des Absorptionskoeffizienten der entsprechenden Struktur nicht notwendig, welche bei einer Segmentierung anhand eines vorgegebenen Schwellenwertes hingegen notwendig ist. Zweckmäßigerweise entsprechen dabei die (segmentierten) Bereiche der entsprechenden anatomischen Struktur bzw. deren Form. Beispielsweise alternativ wird der Bereich in Abhängigkeit der Struktur oder der Art der Struktur größer oder kleiner dimensioniert.

Dabei definiert jede der Bildmasken einen Wirkungsbereich des Initialbildes für eine Abbildungsvorschrift, welche dieser Bildmaske zugeordnet ist. Somit wirkt die Abbildungsvorschrift lediglich in dem der entsprechenden Bildmaske zugeordneten, also von der Bildmaske abgedeckten, Bereich des Initialbildes. Vorzugsweise sind die Bildmasken zueinander disjunkt, d.h. sie weisen keine gemeinsamen Wirkungsbereiche auf und überschneiden sich nicht.

Eine Abbildungsvorschrift, auch als Verfahrensvorschrift oder Abbildungsfunktion bezeichnet, ist oder umfasst dabei insbesondere eine mathematische Funktion. Vorzugsweise basiert diese mathematische Funktion auf einem physikalischen oder statistischen Modell oder wird aus diesem Modell abgeleitet. Die Bildwerte des Initialbildes im entsprechenden Wirkungsbereich bilden dabei die unabhängige Variable der der mathematischen Funktion. Mit anderen Worten werden die Bildwerte mittels der Abbildungsvorschrift bearbeitet, also bestimmt und gegebenenfalls geändert. Dabei ist die Abbildungsfunktion beispielsweise eine abschnittsweise lineare Funktion, welche auf einem Teilintervall oder mehreren Teilintervallen des positiven Werteraums definiert ist. Alternativ ist oder umfasst die Abbildungsfunktion eine Faltung oder eine Filterfunktion zur Bearbeitung der Bildwerte, mittels welcher Filterfunktion Bildqualitätsparameter der Bilddaten, wie beispielsweise ein Rauschen oder eine Bildschärfe, entsprechend bearbeitet werden.

Alternativ oder zusätzlich ist die Abbildungsvorschrift eine insbesondere mathematische Operation, beispielsweise eine Zuordnung der Bildwerte oder eines Anteils des Bildwertes zu einer Klasse, insbesondere einem (Basis-)Material.

Beispielsweise werden mehrere Abbildungsvorschriften auf einen Bereich angewandt.

Anschließend werden verfahrensgemäß die Abbildungsvorschriften in den durch die entsprechende Bildmaske definierten Wirkungsbereichen angewandt. Mit anderen Worten werden die mittels der Bildmasken definierten Wirkungsbereiche mittels der jeweils zugeordneten Abbildungsfunktion bearbeitet, was im Folgenden auch als "maskieren" bezeichnet wird.

In einem weiteren Schritt werden die Bilddaten anhand des mittels der Abbildungsvorschriften derart bearbeiteten Initialbildes erzeugt.

Vorteilhafterweise ist eine Segmentierung des Initialbildes bei Anwesenheit von Artefakten anhand anatomischer Merkmale vergleichsweise wenig fehlerbehaftet, insbesondere im Vergleich zu einer Segmentierung anhand eines Schwellenwertes. Vorteilhaft ist folglich mittels des anhand anatomischer Merkmale segmentierten Initialbildes bzw. mittels der entsprechenden Bereiche, im Folgenden auch kurz als segmentierte Bereiche bezeichnet, eine artefaktfreie oder zumindest artefaktreduzierte Ermittlung und/oder Erzeugung der Bilddaten realisiert.

So werden beispielsweise mittels der Anwendung der Abbildungsfunktionen auf die entsprechenden Bereiche ein Knochenbild aus dem Initialbild extrahiert, indem lediglich Bereiche, welchem dem anatomischen Merkmal des entsprechenden Knochens zugeordnet sind, aus dem Initialbild extrahiert und als Bilddaten herangezogen.

Gemäß einer vorteilhaften Weiterbildung wird den segmentieren Bereichen entsprechend der jeweiligen anatomischen Merkmale jeweils eine Mehr-Materialien-Basis mit einer entsprechenden Anzahl an (Basis-)Materialien zugeordnet. So wird hierbei eine räumliche Verteilung eines Absorptionskoeffizienten (Schwächungskoeffizienten), welche insbesondere mittels der Bildwerte des Initialbildes repräsentiert ist, als ein linearer Zusammenhang, also mittels einer Summe von Produkten, dargestellt. Jedes der Produkte weist dabei als einen Faktor die räumliche Verteilung des Beitrags des jeweiligen Basismaterials zum dieser räumlichen Verteilung des Absorptionskoeffizienten, und als anderen Faktor den von der Energie der Röntgenstrahlung abhängigen (linearen) Absorptionskoeffizienten des entsprechenden Basismaterials auf. Mit anderen Worten weisen die Produkte für jeweils eines der Materialien einen deren räumliche Verteilung darstellenden Faktor und einen deren energieabhängigen Absorptionskoeffizienten darstellenden Faktor auf.

Eine eindeutige Bestimmung der räumlichen Verteilung der Beiträge der Basismaterialien zur räumlichen Verteilung lediglich anhand der Verteilung des Absorptionskoeffizienten, welche insbesondere mittels der Bildwerte des Initialbildes repräsentierten ist, ist dabei im Allgemeinen nicht möglich. Beispielsweise ist für einen gegebenen Bildwert nicht eindeutig bestimmbar, ob dem Bildwert das Material Knochen oder die Materialien Weichgewebe und Kontrastmittel zugrunde liegen bzw. zu welchen Anteilen das jeweilige Material zum Bildwert beiträgt.

Insbesondere bewirkt eine Dichteänderung in der anatomischen Struktur bei gleichbleibendem (Zusammensetzungs-, Dichte-) Verhältnis der Basismaterialien in dieser Struktur, und somit insbesondere auch bei gleichbleibendem Verhältnis der räumlichen Verteilung der Beiträge der entsprechenden Materialien zur räumlichen Verteilung des Absorptionskoeffizienten, eine proportionale Änderung des Absorptionskoeffizienten und eine entsprechende Änderung der Bildwerte. In einer anderen anatomischen Struktur mit diesen Materialien, aber in einem anderen Verhältnis der räumlichen Verteilung der Beiträge der Materialien zur räumlichen Verteilung des Absorptionskoeffizienten, erfolgt bei analoger Dichteänderung eine proportionale Änderung des Absorptionskoeffizienten, allerdings mit einem anderen Proportionalitätsfaktor. Folglich unterscheiden sich bei analoger Dichteänderung die Änderungen des Absorptionskoeffizienten der beiden anatomischen Strukturen. Insbesondere wird aufgrund dessen einer anatomische Struktur eine geeignete Mehr-Materialien-Basis zugeordnet und entsprechend eine geeignete Abbildungsvorschrift gewählt.

Zusammenfassend kann für eine vorgegebene räumliche Verteilung des Absorptionskoeffizienten die räumlichen Verteilung der Beiträge der Basismaterialien nicht eindeutig bestimmt werden, es sind mehrere (Zusammensetzungs-, Dichte-) Verhältnisse möglich.

Mittels der Segmentierung anhand anatomischer Merkmale wird der Raum (eine Anzahl) aller möglichen Verhältnisse der räumlichen Verteilungen der Beiträge der entsprechenden Basismaterialien zum jeweiligen Bildwert, welche der insbesondere mittels der Bildwerte repräsentierten und aus den CT-Rohdaten bestimmten räumlichen Verteilung des Absorptionskoeffizienten entspricht, vergleichsweise stark eingeschränkt oder die räumliche Verteilung der Beiträge kann sogar exakt bestimmt werden. Insbesondere wird somit mittels der Segmentierung anhand anatomischer Merkmale der Raum aller für die Verteilung des Absorptionskoeffizienten möglichen (Zusammensetzungs-, Dichte-) Verhältnisse der Materialien des entsprechend zugeordneten Bereichs (verkleinert) eingeschränkt oder sogar exakt bestimmt. Mittels der derart bestimmten räumlichen Verteilung der Beiträge der entsprechenden Basismaterialien zum jeweiligen Bildwert ist vorteilhafterweise die räumliche Verteilung dieser Materialien vergleichsweise genau abschätzbar oder sogar exakt bestimmbar.

Für medizinische Anwendungen sind beispielsweise als Mehr-Material-Basis für einen einem Knochen zugeordneten Bereich als ein erstes Material Wasser und als ein zweites Material Knochen geeignet. Für einen einem Weichgewebe zugeordneten Bereich ist eine geeignete Mehr-Material-Basis mittels der Materialien Wasser und Kontrastmittel, insbesondere Iod, gebildet.

Für medizinische Anwendungen ist des Weiteren Fettgewebe als ein Basismaterial geeignet. Ist in einem der segmentierten Bereiche lediglich ein einziges Material vorhanden bzw. sofern diesem Bereich lediglich ein Material zugeordnet wird, wie beispielsweise ein Metall für einen Bereich, welchem ein Implantat als anatomisches Merkmal zugeordnet ist, ist dieses Material als eine Ein-Material-Basis für den entsprechenden Bereich geeignet. Insofern ist unter einer Mehr-Materialien-Basis auch eine Ein-Material-Basis zu verstehen.

Beispielsweise werden diese Materialien hierbei zusätzlich durch geeignet gewählte Materialien, insbesondere mit vorbekannten Eigenschaften, approximiert. So kann Knochen durch Kalzium, oder Weichgewebe durch Wasser approximiert werden. Insbesondere werden mittels einer geeigneten Wahl der Basismaterialien für die Mehr-Materialien-Basis deren physikalische Eigenschaften bei der Bildgebung, beispielsweise deren Abhängigkeit des Schwächungskoeffizienten von der Energie der zur Aufnahme der CT-Rohdaten verwendeten Röntgenstrahlung, sowie die Physiologie der entsprechenden anatomischen Struktur berücksichtigt.

Vorzugsweise ist die Mehr-Materialien-Basis als eine Zwei-Materialien-Basis ausgebildet. Mit anderen Worten erfolgt eine Basismaterialzerlegung anhand zweier Materialien. Aufgrund dessen ist der Raum aller möglichen Verteilungen der Beiträge der Basismaterialien für den entsprechenden Bildwert mittels der Segmentierung anhand anatomischer Merkmale vorteilhafterweise kleiner als bei einer Mehr-Materialien-Basis mit mehr als zwei Basismaterialien.

Die Zuordnung bzw. Bestimmung der Beiträge zu den Bildwerten durch die entsprechenden (Basis-)Materialien erfolgt dabei gemäß einer zweckmäßigen Weiterbildung anhand der Abbildungsfunktion. Beispielsweise wird mittels der Abbildungsfunktion einem Material der Mehr-Materialien-Basis mit zunehmendem Bildwert ein entsprechend linear zunehmender Beitrag zum Bildwert zugeordnet. Zusammenfassend werden die Bildwerte mittels der Abbildungsvorschrift bearbeitet und es erfolgt zusätzlich mittels der Abbildungsvorschrift eine Zuordnung der Beiträge zum dem entsprechenden Bildwert zu den entsprechenden Materialien. Ferner zusammenfassend werden die Bereiche mittels der entsprechenden Abbildungsvorschrift bezüglich der jeweiligen (Basis-)Materialien ausgewertet, welche im entsprechenden Bereich vorhanden sind.

Vorteilhafterweise ist es hiermit ermöglicht, Bilddaten zu ermitteln, welche lediglich die räumliche Verteilung eines der Materialien oder aller Materialien oder einer Teilgruppe der Materialien repräsentieren. Beispielsweise wird mittels einer geeignet gewählten Abbildungsfunktion in einem Bereich, welchem Weichgewebe zugeordnet ist, ein Beitrag zum Bildwert durch das Weichgewebe sowie durch ein Kontrastmittel bestimmt. Daraus wird die räumliche Verteilung des Kontrastmittels ermittelt. Die räumliche Verteilung des Kontrastmittels, insbesondere Iod, wird dann zur Erzeugung der Bilddaten herangezogen, und als sogenanntes Iodbild dargestellt. Alternativ werden Bilddaten anhand derjenigen Beiträge bzw. deren Verteilung erzeugt, welche mittels der Abbildungsvorschrift nicht dem Kontrastmittel zugeordnet wurden, so dass die Bilddaten ein Nativbild, also die Darstellung der räumlichen Verteilung der Schwächungskoeffizienten ohne die entsprechenden Beiträge aufgrund des Kontrastmittels, darstellen. Beispielsweise alternativ wird in analoger Weise ein sogenanntes Wasserbild, welches die räumliche Verteilung des Materials Wasser repräsentiert, erzeugt.

Anhand der ermittelten Verteilung der Materialien sowie bei Kenntnis der Abhängigkeit deren Absorptionskoeffizienten von der Energie einfallender Röntgenstrahlung ist es des Weiteren ermöglicht, Bilddaten zu erzeugen, deren Bildwerte einer CT-Aufnahme mit einer durch einen Benutzer vorgegebenen Energie der, insbesondere monochromatischen, Röntgenstrahlung entsprechen.

Gemäß einer vorteilhaften Ausgestaltung wird die Abbildungsvorschrift in Abhängigkeit des dem entsprechenden Bereich zugeordneten anatomischen Merkmals oder der entsprechend zugeordneten Mehr-Materialien-Basis gewählt. Auf diese Weise können vorteilhafterweise unterschiedliche Abbildungsvorschriften für unterschiedliche Mehr-Materialien-Basen oder anatomische Merkmale gewählt werden. Die Abbildungsvorschrift ist dann abhängig von der dem jeweiligen Bereich zugeordneten Mehr-Materialien-Basis bzw. vom anatomischen Merkmal. Insbesondere erfolgt auf diese Weise eine Zuordnung von Beiträgen zu den entsprechenden Bildwerten zu den entsprechenden (Basis-) Materialien vergleichsweise präzise. Somit ist eine Abschätzung der räumlichen Verteilung der Beiträge der (Basis-) Materialien zur Verteilung der insbesondere mittels der Bildwerte repräsentierten Verteilung des Absorptionskoeffizienten und somit eine entsprechende räumliche Verteilung dieser Materialien weiter verbessert.

Gemäß einer vorteilhaften Weiterbildung des Verfahrens werden alternativ oder zusätzlich zur Bestimmung der Bilddaten aus den maskierten Bereichen in einer der oben dargestellten Varianten aus den maskierten Bereichen, vorzugsweise aus jedem maskiertem Bereich zunächst Korrekturdaten erzeugt. Diese werden anschließend zur Bestimmung korrigierter, insbesondere artefaktreduzierter, Bilddaten herangezogen.

Beispielsweise werden hierzu zunächst die räumlichen Verteilungen der Materialien in einer der oben dargestellten Varianten unter Anwendung der Abbildungsvorschriften und der damit einhergehenden Zuordnungen der jeweiligen Beiträge zu Bildwerten des Initialbildes zum entsprechenden Material bestimmt. Mittels einer Vorwärtsprojektion dieser Verteilungen in den sogenannten Projektionsraum wird für diese Materialien anschließend jeweils eine (effektive) Weglänge für die bei der Aufnahme der CT-Rohdaten verwendete, insbesondere polychromatische, Röntgenstrahlung ermittelt. Anhand der ermittelten Weglängen der Materialien und anhand der CT-Rohdaten wird die effektive Weglänge eines dieser Materialien, insbesondere Wasser, korrigiert oder alternativ bestimmt. Mittels der korrigierten effektiven Weglänge dieses Materials und mittels der effektiven Weglängen der anderen Materialien werden dann, beispielsweise mittels einer konvexen Kombination, Linienintegrale bzw. (künstliche, synthetische) CT-(Projektions-) Daten, auch als korrigierte CT-Rohdaten bezeichnet, bestimmt. Diese werden anschließend unter Erzeugung der Bilddaten in den Bildraum übertragen.

Zusammenfassend sind also aufgrund der ermittelten Verteilung der Materialien entsprechend korrigierten Weglängen ermittelt. Synthetische CT-Daten, die mittels dieser korrigierten Weglängen erstellt und in Bilddaten umgewandelt werden, weisen demnach keine oder zumindest vergleichsweise wenige oder vergleichsweise schwach ausgebildete Artefakte auf.

Die Zuordnung der Mehr-Materialien-Basis zum entsprechenden Bereich bzw. zur entsprechenden anatomischen Struktur sowie die Erzeugung der entsprechenden (korrigierten) Bilddaten erfolgt besonders bevorzugt automatisch. Somit sind Fehler aufgrund einer manuellen Eingabe durch einen Benutzer, beispielsweise bei der Wahl der Basismaterialien, vermieden und Artefakte, insbesondere Strahlaufhärtungsartefakte, aufgrund der automatischen Wahl der Basismaterialien und der entsprechend erzeugten Korrekturdaten vermieden oder zumindest verringert.

Gemäß einer geeigneten Ausgestaltung des Verfahrens erfolgt die Bestimmung der korrigierten Bilddaten mittels der erzeugten Korrekturdaten iterativ. Mit anderen Worten werden die korrigierten Bilddaten unter Anwendung der Korrekturdaten mittels einer Iteration ermittelt bzw. erzeugt.

Gemäß einer ersten Variante der iterativen Bestimmung der korrigierten Bilddaten wird eine sogenannte iterative Rekonstruktion verwendet. Insbesondere erfolgt dabei eine im Zuge der iterativen Rekonstruktion angewandte Regularisierung in Abhängigkeit der Korrekturdaten. Hierbei repräsentieren die Korrekturdaten beispielsweise die Zuordnung zu einem segmentierten Bereich bzw. zur zugeordneten anatomischen Struktur oder zu einem der Materialien der Mehr-Materialien-Basis. Die Regularisierung erfolgt dann in Abhängigkeit dieser Zuordnung, insbesondere wird in Abhängigkeit der Zuordnung eine entsprechende mathematische Funktion für die Regularisierung gewählt. Hierbei wird beispielsweise mittels der Regularisierung die Bildschärfe in einem der anatomischen Struktur "Knochen" zugeordneten Bereich erhöht und in einem der anatomischen Struktur "Wasser" bzw. "Weichgewebe" zugeordnetem Bereich ein Bildrauschen verringert.

Alternativ bildet gemäß einer zweiten Variante der iterativen Bestimmung der korrigierten Bilddaten das oben beschriebene Verfahren zur Erzeugung von Bilddaten oder Teilschritte dieses Verfahrens einen Iterationsschritt der Iteration. Hierbei umfasst der Iterationsschritt (Iterationsschleife, Iterationsdurchgang) der Iteration beispielsweise die Segmentierung anhand anatomischer Merkmale, die Maskierung des Initialbildes dieses Iterationsschrittes, das Ermitteln von Korrekturdaten und das Anwenden der Korrekturdaten zur Erzeugung von Bilddaten, welche wiederum zur Erstellung eines Initialbildes des nächsten Iterationsschrittes herangezogen werden. Alternativ umfasst ein Iterationsschritt der Iteration lediglich die Maskierung, das Ermitteln von Korrekturdaten und das Anwenden der Korrekturdaten zur Erzeugung des Initialbildes des nächsten Iterationsschrittes, wobei die Bildmasken vor der Iteration mittels der Segmentierung anhand anatomischer Merkmale bestimmt wurden und diese Masken für alle Iterationsschritte verwendet werden. Die Iteration umfasst also mindestens einen, vorzugsweise mehrere Iterationsschritte. Als Abbruchbedingung für die Iteration dient beispielsweise die Anzahl der Iterationsschritte oder ein Ähnlichkeitsmaß zwischen einem vorgegebenen Modell der anatomischen Struktur und der anatomischen Struktur gemäß den Bilddaten.

Zweckmäßigerweise wird gemäß einer Variante des Verfahrens mittels der Korrekturdaten und der CT-Rohdaten eine Elektronendichteverteilung, also eine räumliche Verteilung der Elektronendichte, als Bilddaten bestimmt. Insbesondere wird die räumliche Verteilung der Elektronendichte zur Planung einer Strahlentherapie verwendet. Hierzu werden beispielsweise die einem Absorptionskoeffizienten entsprechenden Bildwerte, welche beispielsweise in der Hounsfield-Skala dargestellt sind, mittels einer vorbekannten Funktion oder mittels einer vorbekannten Umrechnungstabelle in eine Elektronendichte umgewandelt. Hierbei werden vorzugsweise die Bildwerte der mittels der Korrekturdaten korrigierten CT-Rohdaten umgewandelt. Analog kann dieses Vorgehen zur Bestimmung der räumlichen Verteilung einer Kernladungszahl angewandt werden.

Insbesondere aufgrund der Segmentierung anhand anatomischer Merkmale sowie der, vorzugsweise von der Wahl der zugeordneten Mehr-Materialien-Basis abhängigen, Bearbeitung mittels der Abbildungsfunktion ist vorteilhafterweise eine artefaktfreie oder zumindest artefaktreduzierte räumliche Verteilung der Elektronendichte bestimmbar und auch bestimmt. Somit ist eine verbesserte Bestrahlungsplanung, insbesondere eine Bestimmung einer Verteilung einer in den Patienten eingebrachten Strahlendosis, ermöglicht.

Gemäß einer geeigneten Ausführung wird zur Segmentierung des Initialbildes ein (künstliches) neuronales Netz herangezogen. Vorzugsweise ist dabei das neuronale Netz ein sogenanntes "convolutional neural network", insbesondere ein "deep convolutional neural network". Solche neuronalen Netze werden trainiert und erkennen verallgemeinerte Zusammenhänge oder Muster. Mit anderen Worten werden die zum Training des neuronalen Netzes verwendeten Daten nicht lediglich auswendig gelernt. Infolge dessen sind solche neuronale Netze vorteilhaft vergleichsweise robust gegenüber anatomischen Variationen und/oder einer Variation in der Anreicherung von Iod im zugeordneten Gewebe, d.h. auch bei derartigen Variationen und/oder bei Artefakten im Initialbild erfolgt eine zuverlässige und korrekte Segmentierung des Initialbildes anhand der anatomischen Merkmale. Zum Training des neuronalen Netzes werden beispielsweise CT-Rohdaten oder Bilddaten aus tatsächlich durchgeführten medizinischen CT-Untersuchungen verwendet. Alternativ oder zusätzlich werden mittels einer Simulation erzeugte Daten verwendet. Beispielsweise werden diese Daten in einer Simulation erstellt. Insbesondere werden hierzu Kontrastanreicherungen in vorbekannten Initialbilder errechnet, wobei die Segmentierung anhand anatomischer Merkmale der Initialbilder vorbekannt ist. Beispielsweise ist ein solches neuronales Netz darauf trainiert, anhand der anatomischen Merkmale zwischen Knochen und Weichgewebe zu unterscheiden, insbesondere unabhängig von einem gegebenenfalls vorhandenen Kontrastmittel, und entsprechend das Initialbild in Bereiche zu segmentieren. Zusätzlich oder alternativ ist ein solches neuronales Netz darauf trainiert kontrastmittelangereicherte Gefäße von Knochen für die entsprechende Segmentierung zu unterscheiden. Des Weiteren zusätzlich oder alternativ ist ein solches neuronales Netz darauf trainiert Implantate anhand deren Form und/oder Lage zu erkennen. Aufgrund der Segmentierung solcher Implantate ist es ermöglicht Korrekturdaten zu erzeugen, mittels welcher Metallartefakte aufgrund des Implantats reduziert oder verhindert sind.

Alternativ zur Verwendung eines neuronalen Netzes wird ein Modell-basiertes Verfahren herangezogen, bei welchem ein Volumenmodell der zu segmentierenden Struktur, beispielsweise eines Organs oder eines Knochens, entsprechend der gemessenen Bildwerte angepasst wird. Hierbei erfolgt die Anpassung des Volumenmodells insbesondere in Abhängigkeit einer Größe, welche eine Ähnlichkeit oder Korrelation zwischen dem Volumenmodell, der der Segmentierung zugrunde liegenden anatomischen Struktur und/oder der Bildwerte repräsentiert.

Gemäß einer zweckmäßigen Ausgestaltung werden die CT-Rohdaten in Form von Projektionsbilddaten, bereitgestellt. Die Projektionsbilddaten weisen dabei ein Sinogramm auf. Dieses repräsentiert einen Intensitätsverlauf von auf einen (Röntgen-) Detektor einfallende Röntgenstrahlung für einen entsprechenden (Aufnahme-) Winkel. Vorzugsweise zusätzlich weisen die Projektionsbilddaten Informationen über eine Aufnahmegeometrie, also über eine relative Anordnung zwischen dem Detektor, einer die Röntgenstrahlung erzeugende (Röntgen-) Quelle sowie des zu untersuchenden Objekts, insbesondere des Aufnahmewinkels. Die Projektionsbilddaten werden insbesondere mittels des Computertomographiegeräts erfasst und bereitgestellt. Alternativ werden die Projektionsbilddaten aus einer Datenbank geladen. Sind in einer Datenbank lediglich bereits rekonstruierte Bilddaten hinterlegt, können diese zur Durchführung des Verfahrens, und somit vorteilhafterweise zur Vermeidung von Artefakten, mittels einer Vorwärtsprojektion in CT-Rohdaten übersetzt werden.

Zusammenfassend ist es mittels einer geeigneten Wahl der Abbildungsvorschrift ermöglicht, Bilddaten entsprechend einem vorgegebenen Zweck zu erzeugen. So können die Bilddaten beispielsweise eine räumlichen Verteilung eines Kontrastmittels, eines Absorptionskoeffizienten oder einer Ladungsdichte, beispielsweise der Kernladungszahl oder insbesondere der Elektronendichte repräsentieren. Alternativ oder zusätzlich ist es mittels einer geeigneten Wahl der Abbildungsvorschrift ermöglicht, einen Bildqualitätsparameter, wie etwa ein Bildrauschen, in den entsprechenden Bereichen gezielt zu ändern.

Geeigneter Weise weist ein Bilderzeugungsrechner (Rekonstruktionsrechner) einen Controller auf, welcher dazu eingerichtet ist, das vorstehend beschriebene Verfahren durchzuführen. Der Controller ist beispielsweise ein FPGA (field programmable gate array) oder ein ASIC (application specific integrated circuit) oder weist einen solchen FPGA oder ASIC auf. Zweckmäßigerweise weist der Bilderzeugungsrechner ferner ein computerlesbares Medium (computerlesbarer Datenträger) auf oder zusätzlich oder alternativ ist das computerlesbare Medium mit dem Bilderzeugungsrechner, insbesondere datenübertragungstechnisch, koppelbar. Allenfalls umfasst das computerlesbare Medium ein Computerprogammprodukt, welches einen als Bilderzeugungsprogramm ausgebildeten Programmcode aufweist, bei dessen Ausführung mittels des Controllers das Verfahren in einer der oben beschriebenen Varianten durchgeführt wird. Beispielsweise ist das neuronale Netz zur Durchführung der Segmentierung ein Bestandteil des Bilderzeugungsprogramms. Alternativ weist das Bilderzeugungsprogramm eine Schnittstelle zum Neuronalen Netz auf. Zusammenfassend ist der Bilderzeugungsrechner also dazu eingerichtet, aus bereitgestellten CT-Bilddaten ein Initialbild zu erstellen, dieses zu segmentieren und zu maskieren, um Bilddaten zu erzeugen.

In einer vorteilhaften Ausführung umfasst ein Computertomographiegerät den vorstehend beschriebenen Bilderzeugungsrechner. Der Bilderzeugungsrechner sowie das Computertomographiegerät weisen somit die sich aus dem vorstehend beschriebenen Verfahren ergebenden Merkmale und Vorteile in analoger Weise auf.

Zusammenfassend weist das erfindungsgemäße Computerprogrammprodukt, insbesondere das vorstehend beschriebene Bilderzeugungsprogramm, also Programmcode (d. h. insbesondere entsprechende Instruktionen) zur Durchführung des vorstehend beschriebenen Verfahrens auf, wenn das Computerprogrammprodukt auf einem Computer (insbesondere auf dem vorstehen beschriebenen Bilderzeugungsrechner bzw. mittels dessen Controller) ausgeführt wird. Das erfindungsgemäße Computerprogrammprodukt ist dabei auf dem erfindungsgemäßen Datenträger gespeichert und somit von diesem umfasst.

Nachfolgend werden Ausführungsbeispiele der Erfindung anhand einer Zeichnung näher erläutert. Darin zeigen:
- FIG 1: in einer schematischen Frontalansicht ein Computertomographiegerät mit einem Bilderzeugungsrechner,
- FIG 2: in einem schematischen Ablaufdiagramm ein Verfahren zur Erzeugung von Bilddaten aus CT-Rohdaten, das mit dem Computertomographiegerät durchgeführt wird, wobei ein aus den CT-Rohdaten erstelltes Initialbild mittels Abbildungsvorschriften bearbeitet wird, und
- FIG 3: In einem schematischen Ablaufdiagramm ein alternatives Ausführungsbeispiel des Verfahrens der FIG 2, wobei aus dem bearbeiteten Initialbild Korrekturdaten zur Erzeugung der Bilddaten ermittelt werden.

Nachfolgend werden Ausführungsbeispiele der Erfindung anhand einer Zeichnung näher erläutert. Darin zeigen:

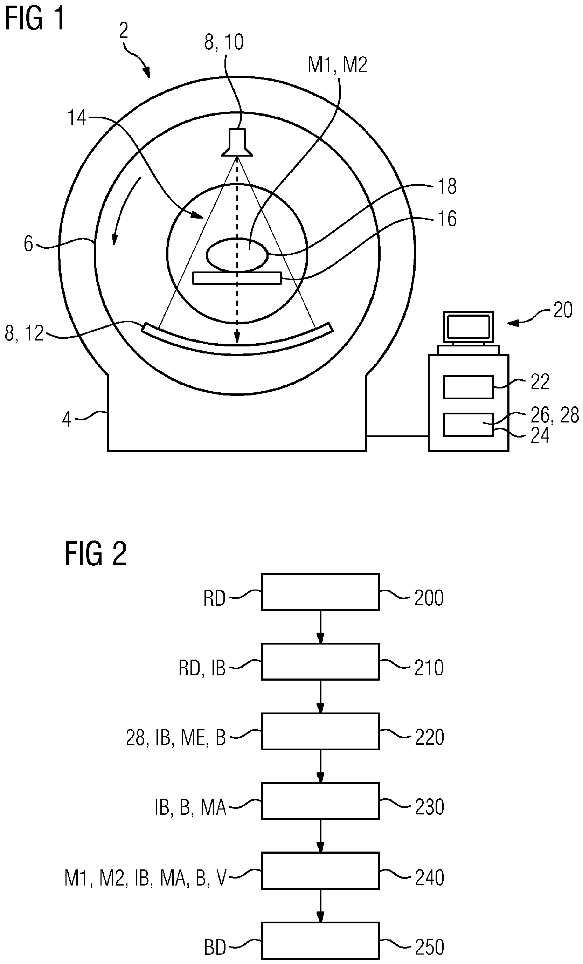

In FIG 1 ist ein Computertomographiegerät 2 gezeigt. Dieses weist einen Halterahmen 4 für einen ringförmigen Drehkranz 6 auf, welcher auch als Gantry bezeichnet wird. Dabei ist der Drehkranz 6 drehbar am Halterahmen gelagert 4. Der Drehkranz 6 trägt ein Röntgensystem 8 mit einer Röntgenquelle 10 zur Erzeugung von Röntgenstrahlung und mit einem in Gegenüberstellung zu dieser angeordneten (Röntgen-) Detektor 12 für die Röntgenstrahlen. Dabei weist die mittels der Röntgenquelle 10 erzeugte Röntgenstrahlung eine spektrale Verteilung auf. Mit anderen Worten ist die Röntgenstrahlung polychromatisch.

In den mittels des ringförmigen Drehkranzes 6 gebildeten, tunnelförmigen Aufnahmebereich 14 (Untersuchungstunnel) ist eine Patientenliege 16 ein- oder verfahrbar. Für das Erfassen (Aufnahme) von CT-Rohdaten RD wird ein zu untersuchendes Objekt 18, insbesondere ein Patient, auf der Patientenliege 16 platziert und in den Aufnahmebereich 14 verfahren. Bei einer Drehung des Drehkranz 6 das sich auf der Patientenliege 16 befindende Objekt 18 kreisförmig vom Röntgensystem 8 umfahren. Folglich werden die CT-Rohdaten RD des Objekts 18 aus unterschiedlichen (Raum-, Aufnahme-) Winkeln aufgenommen, wobei die CT-Rohdaten RD einen Intensitätsverlauf der einfallenden Röntgenstrahlung bei den entsprechenden (Aufnahme-) Winkeln bzw. für die entsprechende Aufnahmegeometrie repräsentieren. Die CT-Rohdaten RD sind derart als Projektionsbilddaten ausgebildet.

Des Weiteren weist das Objekt 18 bzw. der Patient im untersuchten, d.h. mittels der CT-Rohdaten RD erfassten, Bereich (Untersuchungsbereich), unterschiedliche anatomische Strukturen, beispielsweise ein Organ, einen Knochen oder Fettgewebe auf.

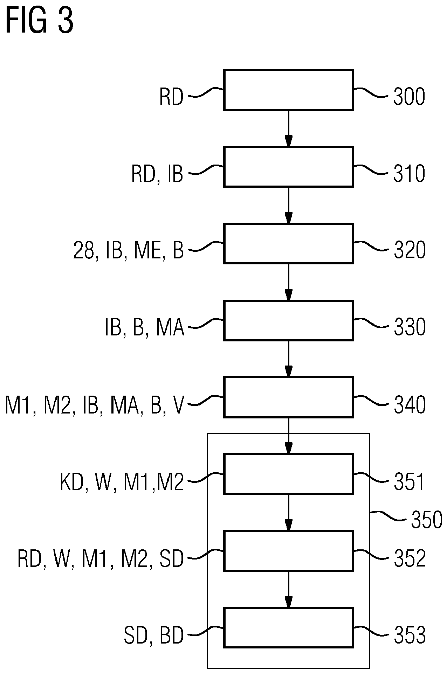

Zur Erfassung des mittels des Detektors 12 erfassten CT-Rohdaten RD sowie zur Steuerung des Röntgensystems 8 ist dieses mit einem Rekonstruktionsrechner 20 signalübertragungstechnisch verbunden. Der Rekonstruktionsrechner 20 weist dabei einen Controller 22 sowie ein als Festplatte ausgebildetes computerlesbares Medium 24 (einen computerlesbaren Datenträger 24) auf, auf welchem ein Computerprogrammprodukt 26 mit einem Bilderzeugungsprogramm mit einem neuronalen Netz 28 hinterlegt ist. Bei Ausführung des Bilderzeugungsprogramms mittels des Controllers 22 wird ein in FIG 2 bzw. FIG 3 gezeigtes und im Nachfolgenden beschriebenes Verfahren zur Erzeugung von Bilddaten BD durchgeführt.

Die FIG 2 zeigt eine erste Ausgestaltung des Verfahrens zur Erzeugung von Bilddaten BD. Hierin werden in einem ersten Schritt 200 die als Projektionsbilddaten ausgebildeten CT-Rohdaten RD bereitgestellt. In einem darauf folgenden zweiten Schritt 210 wird ein Initialbild IB aus den CT-Rohdaten RD, insbesondere mittels der gefilterten Rückprojektion erstellt.

In einem dritten Schritt 220 wird das Initialbild IB anhand anatomischer Merkmale ME in Bereiche B segmentiert. Insbesondere werde als anatomische Merkmale ME eine anatomische Struktur, beispielsweise ein Organ wie die Leber oder ein Knochen, dessen Anordnung relativ zu einem weiteren anatomischen Merkmal, oder dessen Form herangezogen. Dabei entsprechen die Bereiche B der jeweiligen anatomischen Struktur bzw. deren Form. Zur Segmentierung wird ein neuronales Netz 28, insbesondere ein sogenanntes "convolutional neural network", vorzugweise ein "deep convolutional neural network" herangezogen. Dieses ist beispielsweise darauf trainiert, anhand der anatomischen Merkmale ME zwischen Knochen und Weichgewebe zu unterscheiden, insbesondere unabhängig von einem gegebenenfalls vorhandenen Kontrastmittel, und entsprechend das Initialbild IB in Bereiche B zu segmentieren.

In einem anschließenden vierten Schritt 230 wird für das Initialbild IB entsprechend der Bereiche B jeweils eine Bildmaske MA, auch als Maske MA bezeichnet, erstellt. Diese definieren jeweils einen Wirkungsbereich des Initialbildes IB, in welchem eine der Bildmaske MA zugeordnete (Abbildungsfunktion) Abbildungsvorschrift V wirkt, also angewendet wird.

Im fünften Schritt 240 werden die Abbildungsvorschriften V in den mittels der Bildmasken MA definierten Wirkungsbereichen angewandt. Hierbei werden den (segmentierten) Bereichen B entsprechend der jeweiligen anatomischen Merkmale ME jeweils eine Zwei-Materialien-Basis mit den (Basis-) Materialien M1 und M2 zugeordnet. Das Initialbild IB wird hier in zwei Bereiche B aufgeteilt. Der zweite Bereich B entspricht dabei der anatomischen Struktur eines Knochens des Patienten und der erste Bereich B entspricht der anatomischen Struktur eines den Knochen umgebenden Weichgewebes. Ferner wird dem ersten der beiden Bereiche B eine als Zwei-Materialien-Basis ausgebildete Mehr-Materialien-Basis mit den (Basis-) Materialien M1 und M2 Kontrastmittel bzw. Weichgewebe, welches durch Wasser approximiert ist, zugeordnet. So ist bei der Wahl der Zwei-Materialien-Basis berücksichtigt, dass Knochen als weiteres Material definitionsgemäß in einer anatomischen Struktur "Weichgewebe" nicht vorhanden ist. Dem zweiten der beiden Bereiche B wird eine als Zwei-Materialien-Basis ausgebildete Zwei-Materialien-Basis mit den Materialien M1 und M2 Knochen bzw. Wasser. zugeordnet. Hierbei wird im zweiten Bereich B ein Beitrag aufgrund eines Kontrastmittels zu Null angenommen, da dieses typischerweise lediglich in vernachlässigbarer Konzentration in der anatomischen Struktur "Knochen" auftritt.

Die Bildwerte der Bereiche B des Initialbildes IB werden mittels der jeweiligen Abbildungsvorschrift V bezüglich der entsprechenden Materialien M1 und M2 ausgewertet, mit anderen Worten werden die Beiträge der Materialien M1 und M2 zum entsprechenden Bildwert des Initialbildes IB bestimmt. Die Abbildungsvorschriften V sind dabei abhängig der jeweiligen Zwei-Materialien-Basis, d.h. die beiden Bereiche B werden mittels der entsprechenden Abbildungsvorschrift V unterschiedlich bearbeitet. Die Abbildungsvorschriften V sind dabei mathematische Funktionen, welche den Bildwerten als freie Variable der Funktion jeweils die Beiträge zu diesem Bildwert durch die Materialien M1 und M2 bestimmt, und eine damit verknüpfte mathematische Operation, welche die bestimmten Beiträge dem entsprechenden Material M1 bzw. M2 des entsprechenden Bereichs B zuordnet. Mittels dieser Zuordnung und den bestimmten Beiträgen wird dann eine Verteilung der Materialien M1 und M2 in den jeweiligen Bereichen B bestimmt.

Gemäß diesem Ausführungsbeispiel unterscheiden sich die Abbildungsvorschriften V dabei in Abhängigkeit der jeweils zugeordneten Zwei-Materialien-Basis. So werden für den ersten Bereich B die Beiträge zum Bildwert aufgrund vom Kontrastmittel als Material M1 und aufgrund von Wasser als Material M2 bestimmt und zugeordnet. Ein Beitrag aufgrund von Knochen als weiteres Material in diesem Bereich B wird entsprechend der gewählten Zwei-Materialien-Basis stets zu Null angenommen und somit mittels der Abbildungsfunktion V kein Beitrag zum Bildwert aufgrund von Knochen zugeordnet. Für den zweiten Bereich B werden analog die Beiträge zu den Bildwerten aufgrund von Knochen als Material M1 und aufgrund von Wasser als Material M2 bestimmt und entsprechend zugeordnet. Ein Beitrag aufgrund eines Kontrastmittels wird entsprechend der gewählten Zwei-Materialien-Basis zu Null angenommen. Mittels der Bestimmung des Beitrags zu den Bildwerten und der entsprechenden Zuordnung zum Material Kontrastmittel, sowie anhand der bekannten Position des dem entsprechenden Bildwert zugeordnetem Pixel oder Voxel, wird eine räumliche Verteilung des Kontrastmittels bestimmt. Aus dieser Verteilung werden in einem sechsten Schritt 250 die Bilddaten BD erzeugt. Hier sind die Bilddaten BD als ein Kontrastmittelbild ausgebildet, welches die räumliche Verteilung des Kontrastmittels im untersuchten Bereich des Objekts 18 darstellt. Zusätzlich oder alternativ sind in analoger Weise die Bilddaten BD als ein Knochenbild, also die mittels der Abbildungsvorschrift bestimmte räumliche Verteilung des Materials Knochen, ausgebildet. Gemäß dieser Variante des Verfahrens werden zusammenfassend also Bilddaten BD erzeugt, welche die Verteilung eines der Materialien M1 oder M2 zeigen.

In einer nicht weiter dargestellten Alternative des Verfahrens gemäß der FIG 2 werden im fünften Schritt 240 die Abbildungsvorschriften V in den mittels der Bildmasken MA definierten Wirkungsbereichen angewandt, wobei die anderen Schritte analog durchgeführt werden. Die Abbildungsvorschrift V ist hier nicht abhängig vom entsprechenden Bereich zugeordneten anatomischen Merkmals ME oder der entsprechend zugeordneten Mehr-Materialien-Basis. Beispielsweise ist die Abbildungsvorschrift V eine Filterfunktion, welchen Bildwerten unterhalb eines Schwellenwertes den Wert Null zuordnet. In alternativer Ausgestaltung wird mittels der Abbildungsvorschrift ein Rauschen reduziert oder eine Bildschärfe erhöht. Allenfalls werden mittels dieser Filterfunktion die entsprechenden Wirkungsbereiche bearbeitet und aus dem derart bearbeiteten Initialbild IB die Bilddaten BD im sechsten Schritt 250 erzeugt.

Die FIG 3 zeigt eine weitere alternative Ausgestaltung des Verfahrens zur Erzeugung von Bilddaten BD. Die Schritte 300 bis einschließlich 340 erfolgen dabei analog zu den Schritten 200 bis 240 der Ausführung der FIG 2 und werden daher nicht weiter beschrieben. Mittels der im Schritt 340 bestimmten Verteilung der Materialien M1 und M2, hier Wasser Knochen und Iod als Kontrastmittel, werden zunächst Korrekturdaten KD (Teilschritt 351 des Schritts 350) erzeugt. Gemäß diesem Ausführungsbeispiels werden hierzu für jedes der Materialien M1 bzw. M2 dessen räumliche Verteilung in allen Bereichen B mittels einer Vorwärtsprojektion in den sogenannten Projektionsraum übertragen und für jedes der Materialien M1 und M2 jeweils (effektive) Weglängen W der bei der Aufnahme der CT-Rohdaten RD verwendeten polychromatische Röntgenstrahlung und der entsprechenden Aufnahmegeometrie ermittelt.

Anhand der ermittelten Weglängen W aller Materialien M1 und M2 und anhand der CT-Rohdaten RD wird die effektive Weglänge W eines Materials M1 oder M2 korrigiert oder ermittelt (Teilschritt 352 des Schritts 350). Hier werden mittels der CT-Rohdaten sowie mittels der bestimmten Weglängen W der Materialien Knochen (Material M1 im ersten Bereich) und Kontrastmittel (Material M1 im ersten Bereich) die Weglänge W des Materials Wasser (Material M1) bestimmt bzw. die in Schritt 351 bestimmte Weglänge W für Wasser (Material M1 für den zweiten Bereich und Material M2 für den ersten Bereich) korrigiert.

Mittels der korrigierten Weglänge W des Materials Wasser sowie mittels der Weglängen W der anderen Materialien M1 und M2, also Kontrastmittel und Knochen, werden mittels einer sogenannten konvexen Kombination synthetische CT-(Projektions-)Daten SD erzeugt. Ferner werden hierbei die Bildwerte mittels einer vorbekannten Funktion oder mittels einer vorbekannten Umrechnungstabelle in eine Elektronendichte umgewandelt. In Teilschritt 353 des Schritts 350 werden aus diesen synthetischen CT-Daten SD die die Elektronendichte repräsentierende Bilddaten BD durch Rückprojektion erzeugt. Zusammenfassend werden gemäß dieser alternativen Ausgestaltung also Bilddaten BD, welche eine korrigierte Elektronendichteverteilung aller Materialien M1 und M2 repräsentieren, erzeugt.

In einer nicht weiter dargestellten Variante des Verfahrens gemäß der FIG 3 erfolgt die Bestimmung bzw. die Erzeugung der korrigierten Bilddaten BD iterativ. Dabei bilden die Schritte 310 bis 352 einen Iterationsschritt, also einen Iterationsdurchlauf, einer Iteration. Die in Teilschritt 352 erzeugten synthetischen CT-Daten SD werden zur Erzeugung des Initialbilds IB des nächsten Iterationsschrittes herangezogen.

In einer weiteren nicht dargestellten Variante des Verfahrens gemäß der FIG 3 erfolgen die Schritte 300 bis 340 analog, lediglich Schritt 350 unterscheidet sich wie im Folgenden dargestellt. In einem ersten Teilschritt 351 des Schritts 350 werden Korrekturdaten KD erzeugt. Die Korrekturdaten KD repräsentieren hier die Zuordnung zu einem segmentierten Bereich B oder zu einem der Materialien M1 oder M2 der Mehr-Materialien-Basis. In einem zweiten Teilschritt 352 des Schritts 350 werden dann die Korrekturdaten KD bei einer iterativen Rekonstruktion verwendet. So erfolgt eine im Zuge der iterativen Rekonstruktion angewandte Regularisierung in Abhängigkeit der Korrekturdaten KD. Die Regularisierung erfolgt also in Abhängigkeit dieser Zuordnung. Mittels der Regularisierung werden bei der Erzeugung der Bilddaten BD als Ergebnis der iterativen Rekonstruktion aufgrund der Korrekturdaten die Bildschärfe in einem der anatomischen Struktur "Knochen" zugeordneten Bereich B erhöht und in einem der anatomischen Struktur "Wasser" bzw. "Weichgewebe" zugeordnetem Bereich B ein Bildrauschen verringert.

Die Erfindung ist nicht auf die vorstehend beschriebenen Ausführungsbeispiele beschränkt. Vielmehr können auch andere Varianten der Erfindung vom Fachmann hieraus abgeleitet werden, ohne den Gegenstand der Erfindung zu verlassen. Insbesondere sind ferner alle im Zusammenhang mit den Ausführungsbeispielen beschriebenen Einzelmerkmale auch auf andere Weise miteinander kombinierbar, ohne den Gegenstand der Erfindung zu verlassen.

## Patentansprüche

1. Verfahren zur Erzeugung von Bilddaten (BD) bei einem Computertomographiegerät (2),
- wobei CT-Rohdaten (RD) bereitgestellt werden,
- wobei ein Initialbild (IB) aus den CT-Rohdaten(RD) erstellt wird,
- wobei das Initialbild (IB) anhand anatomischer Merkmale (ME) in Bereiche (B) segmentiert wird,
- wobei für das Initialbild (IB) entsprechend der Bereiche (B) jeweils eine Bildmaske (MA) erstellt wird, welche einen Wirkungsbereich für eine jeweils zugeordnete Abbildungsvorschrift (V) definiert,
- wobei die Abbildungsvorschriften (V) in den durch die jeweiligen Bildmasken (MA) definierten Wirkungsbereichen angewendet werden, und
- wobei die Bilddaten (BD) anhand des mittels der Abbildungsvorschriften (V) bearbeiteten Initialbildes (IB) erzeugt werden.

2. Verfahren nach Anspruch 1,
wobei den segmentierten Bereichen (B) entsprechend des jeweiligen anatomischen Merkmals (ME) jeweils eine Mehr-Materialien-Basis mit Materialien (M1,M2) zugeordnet wird, und wobei eine räumliche Verteilung der Materialien (M1,M2) der jeweiligen Mehr-Materialien-Basis mittels der entsprechenden Abbildungsvorschrift (V) ermittelt wird.

3. Verfahren nach Anspruch 1 oder 2,
wobei die Abbildungsvorschrift (V) in Abhängigkeit des dem entsprechenden Bereich (B) zugeordneten anatomischen Merkmals (ME) oder der entsprechend zugeordneten Mehr-Materialien-Basis gewählt wird.

4. Verfahren nach Anspruch 2 oder 3,
wobei zur Ermittlung der räumlichen Verteilung der Materialien (M1,M2) der jeweiligen Mehr-Materialien-Basis jeweils ein Beitrag dieser Materialien (M1,M2) zu entsprechenden Bildwerten des Initialbildes (IB) mittels der Abbildungsvorschrift (V) bestimmt wird.

5. Verfahren nach einem der Ansprüche 1 bis 4,
wobei Korrekturdaten (KD) zur Bestimmung korrigierter Bilddaten (BD) anhand der mittels der entsprechenden Abbildungsvorschrift (V) bearbeiteten Bereiche (B) erzeugt werden.

6. Verfahren nach Anspruch 5,
wobei die korrigierten Bilddaten (BD) mittels der Korrekturdaten (KD) iterativ bestimmt werden.

7. Verfahren nach Anspruch 6,
wobei zur Erzeugung der Bilddaten (BD) eine iterative Rekonstruktion herangezogen wird, deren Regularisierung in Abhängigkeit der Korrekturdaten (KD) erfolgt.

8. Verfahren nach Anspruch 6,
wobei ein Iterationsschritt einer Iteration zumindest aus dem Anwenden der Abbildungsvorschriften (V), der Erzeugung der Korrekturdaten (KD) sowie dem Erzeugen korrigierter Bilddaten (BD) gebildet wird.

9. Verfahren nach einem der Ansprüche 5 bis 8,
wobei mittels der Korrekturdaten (KD) und der CT-Rohdaten (RD) eine Elektronendichteverteilung als Bilddaten (BD) bestimmt wird.

10. Verfahren nach einem der Ansprüche 1 bis 9,
wobei zur Segmentierung des Initialbildes (IB) ein Neuronales Netz (28), insbesondere ein deep convolutional neural network, herangezogen wird.

11. Verfahren nach einem der Ansprüche 1 bis 10,
wobei die CT-Rohdaten (RD) in Form von Projektionsbilddaten bereitgestellt werden.

12. Bilderzeugungsrechner (20) mit einem Controller (22) zur Durchführung des Verfahrens nach einem der Ansprüche 1 bis 11.

13. Computertomographiegerät (2) mit einem Bilderzeugungsrechner (20) nach Anspruch 12.

14. Computerprogrammprodukt (26) mit Programmcode zur Durchführung des Verfahrens zur Bilderzeugung nach einem der Ansprüche 1 bis 11, wenn das Computerprogrammprodukt auf einem Computer (20) ausgeführt wird.

15. Computerlesbarer Datenträger (24), der das Computerprogrammprodukt (26) nach Anspruch 14 umfasst.

## Geänderte Patentansprüche

### Geänderte Patentansprüche gemäss Regel 137(2) EPÜ.

1. Verfahren zur Erzeugung von Bilddaten (BD) bei einem Computertomographiegerät (2),
- wobei CT-Rohdaten (RD) bereitgestellt werden,
- wobei ein Initialbild (IB) aus den CT-Rohdaten(RD) erstellt wird,
- wobei das Initialbild (IB) anhand anatomischer Merkmale (ME) in Bereiche (B) segmentiert wird,
- wobei für das Initialbild (IB) entsprechend der Bereiche (B) jeweils eine Bildmaske (MA) erstellt wird, welche einen Wirkungsbereich für eine jeweils zugeordnete Abbildungsvorschrift (V) definiert,
- wobei die Abbildungsvorschriften (V) in den durch die jeweiligen Bildmasken (MA) definierten Wirkungsbereichen angewendet werden, und
- wobei die Bilddaten (BD) anhand des mittels der Abbildungsvorschriften (V) bearbeiteten Initialbildes (IB) erzeugt werden,
**dadurch gekennzeichnet,**
- **dass** den segmentierten Bereichen (B) entsprechend des jeweiligen anatomischen Merkmals (ME) jeweils eine Mehr-Materialien-Basis mit Materialien (M1,M2) zugeordnet wird, und wobei eine räumliche Verteilung der Materialien (M1,M2) der jeweiligen Mehr-Materialien-Basis mittels der entsprechenden Abbildungsvorschrift (V) ermittelt wird,
- wobei zur Ermittlung der räumlichen Verteilung der Materialien (M1,M2) der jeweiligen Mehr-Materialien-Basis jeweils ein Beitrag dieser Materialien (M1,M2) zu entsprechenden Bildwerten des Initialbildes (IB) mittels der Abbildungsvorschrift (V) bestimmt wird, und wobei die räumliche Verteilung zumindest eines der Materialien (M1,M2) zur Erzeugung der Bilddaten (BD) herangezogen wird.

2. Verfahren nach Anspruch 1,
wobei die Abbildungsvorschrift (V) in Abhängigkeit des dem entsprechenden Bereich (B) zugeordneten anatomischen Merkmals (ME) oder der entsprechend zugeordneten Mehr-Materialien-Basis gewählt wird.

3. Verfahren nach Anspruch 1 oder 2,
wobei Korrekturdaten (KD) zur Bestimmung korrigierter Bilddaten (BD) anhand der mittels der entsprechenden Abbildungsvorschrift (V) bearbeiteten Bereiche (B) erzeugt werden.

4. Verfahren nach Anspruch 3,
wobei anhand der räumlichen Verteilung der Materialen (M1,M2) der Mehr-Materialien-Basen durch Vorwärtsprojektion effektive Weglängen (W) ermittelt werden, und wobei die effektive Weglänge (W) eines der Materialien (M1,M2) anhand der effektiven Weglängen (W) der anderen Materialen bestimmt oder korrigiert wird.

5. Verfahren nach Anspruch 3 oder 4,
wobei die korrigierten Bilddaten (BD) mittels der Korrekturdaten (KD) iterativ bestimmt werden.

6. Verfahren nach Anspruch 5,
wobei zur Erzeugung der Bilddaten (BD) eine iterative Rekonstruktion herangezogen wird, deren Regularisierung in Abhängigkeit der Korrekturdaten (KD) erfolgt.

7. Verfahren nach Anspruch 5,
wobei ein Iterationsschritt einer Iteration zumindest aus dem Anwenden der Abbildungsvorschriften (V), der Erzeugung der Korrekturdaten (KD) sowie dem Erzeugen korrigierter Bilddaten (BD) gebildet wird.

8. Verfahren nach einem der Ansprüche 3 bis 7,
wobei mittels der Korrekturdaten (KD) und der CT-Rohdaten (RD) eine Elektronendichteverteilung als Bilddaten (BD) bestimmt wird.

9. Verfahren nach einem der Ansprüche 1 bis 8,
wobei zur Segmentierung des Initialbildes (IB) ein Neuronales Netz (28), insbesondere ein deep convolutional neural network, herangezogen wird.

10. Verfahren nach einem der Ansprüche 1 bis 9,
wobei die CT-Rohdaten (RD) in Form von Projektionsbilddaten bereitgestellt werden.

11. Bilderzeugungsrechner (20) mit einem Controller (22) zur Durchführung des Verfahrens nach einem der Ansprüche 1 bis 10.

12. Computertomographiegerät (2) mit einem Bilderzeugungsrechner (20) nach Anspruch 11.

13. Computerprogrammprodukt (26) mit Programmcode zur Durchführung des Verfahrens zur Bilderzeugung nach einem der Ansprüche 1 bis 10, wenn das Computerprogrammprodukt auf einem Computer (20) ausgeführt wird.

14. Computerlesbarer Datenträger (24), der das Computerprogrammprodukt (26) nach Anspruch 13 umfasst.
